# EUROPEAN PATENT APPLICATION

(11) **EP 1 396 266 A1**
(43) Date of publication of application: **10.03.2004**
(21) Application number: 02733420.0
(22) Date of filing: 07.06.2002
(51) Int. Cl.: A61K 31/4709, A61K 9/08, A61K 47/32, A61K 47/36, A61K 47/40, A61P 1/04, A61P 7/02, A61P 9/00, A61P 9/10, A61P 9/12, A61P 11/06, A61P 29/00, A61P 43/00

(54) **AQUEOUS CILOSTAZOL PREPARATION FOR INJECTION**

(30) Priority: 13.06.2001 JP 2001179239; 26.04.2002 JP 2002127065
(71) Applicant: OTSUKA PHARMACEUTICAL FACTORY, INC., Naruto-shi, Tokushima 772-8601 (JP); OTSUKA PHARMACEUTICAL CO., LTD., Chiyoda-ku Tokyo 101-8535 (JP)
(72) Inventor: INOUE, Fujio, Naruto-shi, Tokushima 772-0041 (JP); KAWAKAMI, Keiichi, Tokushima 771-0204 (JP); NAGAYASU, Shinya, Tokushima-shi, Tokushima 770-0802 (JP); OKAMOTO, Hideshi, Tokushima-shi, Tokushima 771-1155 (JP)
(74) Representative: Teipel, Stephan, Dr.
(86) International application number: PCT/JP2002/005705
(87) International publication number: WO 2002/102383

(57) **Abstract**

Addition of a cyclodextrin compound to the cilostazol aqueous pharmaceutical preparation for parenteral use which comprises cilostazol or a pharmacologically acceptable salt thereof as an active ingredient can increase water solubility of cilostazol or a pharmacologically acceptable salt thereof, giving an aqueous pharmaceutical preparation for parenteral use of cilostazol or a pharmacologically acceptable salt thereof, of which the stability with time in the liquid state is improved.

## Description

### TECHNICAL FIELD

The present invention relates to an aqueous pharmaceutical preparation for parenteral use containing cilostazol or a pharmacologically acceptable salt thereof as active ingredient.

### BACKGROUND OF THE INVENTION

A report on the Therapeutic Evidences recently published by the Health and Welfare Sciences Research Committee of the Ministry of Health, Labor and Welfare says that antiplatelet drugs are the first choice remedies for patients in the acute phase of acute myocardial or cerebral infarctions. However, a serious problem is that all the anti-platelet drugs on the market are for oral administration, which makes it very difficult, for example, to administrate the drugs to patients who are just suffering acute myocardial or cerebral infarctions, especially in the acute phases thereof, and also to expect immediate effect when administered orally.

The reason why all the anti-platelet drug preparations on the market are for oral use is that all the effective ingredients contained in those preparations are difficult to dissolve in water. Cilostazol, which is generally used as anti-platelet drug, is not an exception and hardly dissolves in water, the solubility at ordinary temperatures being as low as about 3 µg/ml.

As cilostazol dissolves only slightly in water, there has ever been almost no description of the aqueous pharmaceutical preparation which contains the drug as the active ingredient, except for the one described in JP-A1-H10-175864, namely an aqueous pharmaceutical preparation for parenteral use which contains cilostazol, as the active ingredient, and polyethylene glycol 4000 and polyoxyethylenesorbitan mono-oleate, but without containing a cyclodextrin compound. However, such aqueous pharmaceutical preparations have not been realistic because reproducibility of such aqueous pharmaceutical preparations are not necessarily well, and, may lead, even if such a pharmaceutical preparation can be obtained, to separation of cilostazol on standing for a while. Thus, a more convenient or useful formulation of the aqueous pharmaceutical preparation containing cilostazol for parenteral use has been desired. The present inventors have conceived to develop a new cilostazol-containing aqueous pharmaceutical preparation, which is ready to use i.e. does not need dissolution at the time of use and is stable even after long storage without depositing precipitates of cilostazol.

### DISCLOSURE OF THE INVENTION

The aim of the present invention is to provide an aqueous pharmaceutical preparation for parenteral use which contains cilostazol or a pharmacologically acceptable salt thereof, and has an improved stability over time in a liquid state, by increasing the solubility of cilostazol or the pharmacologically acceptable salt thereof.

The present inventors have made an intensive study to attain their object and found that addition of a cyclodextrin compound brings about an increase of the water solubility of cilostazol or a pharmacologically acceptable salt thereof to an extent beyond expectation. They have also found that the addition of a cyclodextrin compound not only improves the water solubility of cilostazol or the pharmacologically acceptable salt thereof, but also enhances the stability over time in a dissolved state of the resulting aqueous pharmaceutical preparation for parenteral use which contains the cilostazol compound as active ingredient, thus making it possible to supply not only aqueous pharmaceutical preparation which can be used by dissolving them just before use, but those ready to use. Thus, the aqueous pharmaceutical preparation for parenteral use which the present invention provides makes it possible to solve the problems which come from the fact, as depicted above, that only oral pharmaceutical preparations have been available, and to supply to the patients of, for example, acute myocardial or cerebral infarctions ready to administer and quick-acting aqueous pharmaceutical preparations for parenteral use.

The present inventors have studied further based on the findings said above and completed the present invention.

Thus, the present invention relates to:
(1) A cilostazol aqueous pharmaceutical preparation for parenteral use, which comprises cilostazol or a pharmacologically acceptable salt thereof as an active ingredient and a cyclodextrin compound;
(2) A cilostazol aqueous pharmaceutical preparation for parenteral use described in (1), wherein cilostazol or a pharmacologically acceptable salt thereof and a cyclodextrin compound are dissolved in either water or a solvent containing water;
(3) A cilostazol aqueous pharmaceutical preparation for parenteral use described in (2), wherein the preparation contains cilostazol or a pharmacologically acceptable salt thereof in a concentration from 0.01 to 20 mg/mL;
(4) A cilostazol aqueous pharmaceutical preparation for parenteral use described in (3), wherein it contains 1 to 4000 moles of a cyclodextrin compound per 1 mole of cilostazol;
(5) A cilostazol aqueous pharmaceutical preparation for parenteral use described in (1), wherein the cyclodextrin compound is a sulfoalkylether derivative of cyclodextrin;
(6) A cilostazol aqueous pharmaceutical preparation for parenteral use described in (5), wherein the cyclodextrin sulfoalkylether is in the form of an alkali metal salt thereof;
(7) A cilostazol aqueous pharmaceutical preparation for parenteral use described in (5), wherein the cyclodextrin sulfoalkylether derivative is a sulfobutylether-beta-cyclodextrin alkali-metal salt;
(8) A cilostazol aqueous pharmaceutical preparation for parenteral use described in (5), wherein the cyclodextrin sulfoalkylether is a hepta-substituted sulfobutylether-beta-cyclodextrins alkali-metal salt;
(9) A cilostazol aqueous pharmaceutical preparation for parenteral use described in (8), wherein the preparation contains cilostazol or a pharmacologically acceptable salt thereof in a concentration from 0.01 to 20 mg/mL;
(10) A cilostazol aqueous pharmaceutical preparation for parenteral use described in (9), wherein the preparation contains from 1 to 4000 moles of a cyclodextrin compound per 1 mole of cilostazol;
(11) A cilostazol aqueous pharmaceutical preparation for parenteral use described in (10), wherein the preparation contains cilostazol or a pharmacologically acceptable salt thereof in a concentration of 0.05 to 5 mg/mL and heptasubstituted sulfobutylether-beta-cyclodextrin sodium salt in a concentration of 100 to 400 mg/mL;
(12) A cilostazol aqueous pharmaceutical preparation for parenteral use described in (11), wherein the preparation contains, furthermore, at least one of dissolution auxiliaries selected from dextran and polyvinylpyrrolidone;
(13) Use of a cyclodextrin compound for increasing the water solubility of cilostazol or a pharmacologically acceptable salt thereof;
(14) A symptom ameliorant of an acute myocardial or a cerebral infarction in their acute phases, which comprises the aqueous pharmaceutical preparation containing cilostazol or a pharmacologically acceptable salt therof described in any of (1) to (11);
(15) A symptom ameliorant of an acute myocardial or a cerebral infarction described in (14), which comprises furthermore at least one of dissolution auxiliaries selected from dextran and polyvinylpyrrolidone; and
(16) A method to ameliorate a symptom in the acute phase of acute myocardial or a cerebral infarction, which comprises administering to a patient via vein an aqueous pharmaceutical preparation of cilostazol described in any of (1) to (11).

### BEST MODE FOR CARRYING OUT THE INVENTION

The aqueous pharmaceutical preparation for parenteral use of the present invention contains cilostazol or a pharmacologically acceptable salt thereof as the active ingredient.

Cilostazol is 6-[4-(1-cyclohexyl-1,2,3,4-Tetrazol-5-yl)butoxy]-3,4-dihydrocarbostyril of the chemical structure shown below.

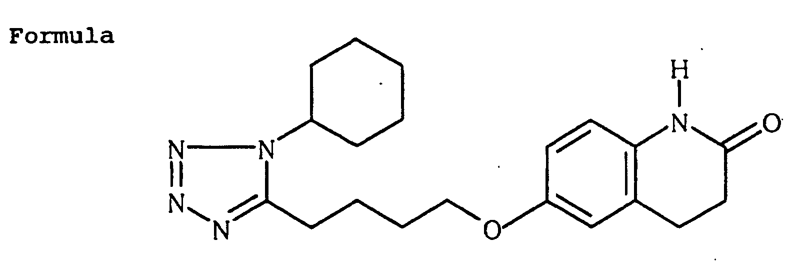

The pharmacologically acceptable salt of cilostazol can be formed readily by allowing cilostazol to react with a pharmacologically acceptable acid. As such pharmacologically acceptable acid, there may be mentioned, for example, an inorganic acid such as hydrochloric acid, sulfuric acid, phosphoric acid, hydrobromic acid, or the like and an organic acid such as oxalic acid, maleic acid, fumaric acid, malic acid, tartaric acid, citric acid, benzoic acid, or the like.

The aqueous pharmaceutical preparation for parenteral use is characterized by that the preparation contains, in addition to the active ingredient mentioned above, a cyclodextrin compound. Here, a cyclodextrin compound means cyclodextrin or a derivative of cyclodextrin or a pharmacologically acceptable salt thereof.

The cyclodextrin compound usable in the present invention includes a cyclic oligosugar comprising 6 to 12 glucose units. And, the derivative of cyclodextrin usable in the present invention includes a compound resulting from substituting a part of or all the hydroxyl groups on the 2, 3 and 6 positions of the glucose units with any other functional group(s). As a specific example of the said cyclodextrin or the derivatives thereof, there may be mentioned a compound represented by the following general formula: 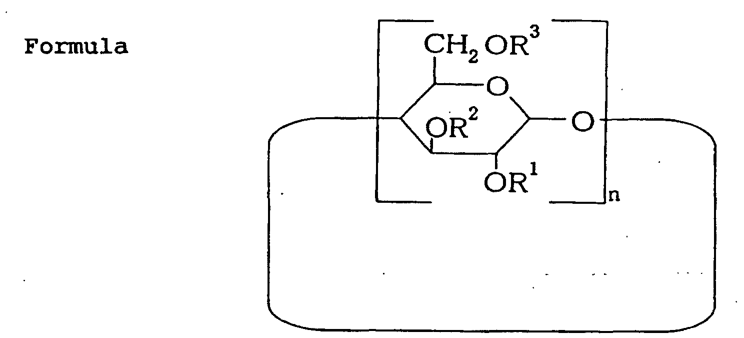 (wherein, n stands for an integer from 6 to 12; each of R¹, R² and R³ in each repeated unit represents independently a hydrogen, an alkyl group, a monohydroxyalkyl group, a dihydroxyalkyl group, a sulfoalkyl group, a carboxyalkyl group or a sugar residue).

In the Formula above, as the alkyl group represented by R¹ to R³, there may be mentioned a C₁₋₄ alkyl group such as, for example, methyl, ethyl, propyl, or the like. As the monohydroxyalkyl group represented by R¹ to R³, there may be mentioned a monohydroxy-C₁₋₄ alkyl group such as, for example, hydroxymethyl, 2-hydroxyethyl, 2-hydroxypropyl or the like. As the dihydroxyalkyl group represented by R¹ to R³, there may be mentioned a dihydroxy-C₁₋₄ alkyl group such as, for example, dihydroxymethyl, 2,2-dihydroxyethyl, dihydroxypropyl or the like. As the sulfoalkyl group represented by R¹ to R³, there may be mentioned a sulfo-C₁₋₄ alkyl group such as, for example, sulfomethyl, 2-sulfoethyl, sulfobutyl or the like. As the carboxyalkyl group represented by R¹ to R³, there may be mentioned a carboxy-C₁₋₄ alkyl group such as, for example, carboxymethyl, 2-carboxyethyl or the like. As the sugar residue represented by R¹ to R³, there may be mentioned, for example, a glucosyl group, a maltosyl group, a panosyl group or the like.

The cyclodextrin compound preferably usable in the present invention is alpha-cyclodextrin or its derivative represented by the general Formula above, wherein n=6; beta-cyclodextrin or its derivative represented by the Formula above, wherein n=7; gamma-cyclodextrin and its derivative represented by the Formula above, wherein n=8; or delta-cyclodextrin and its derivative represented by the Formula above, wherein n=9.

As the more preferable example among the derivative of a cyclodextrin compound described above, there may be mentioned an alkyl derivative, a hydroxyalkyl derivative, a sulfoalkylether derivative, a cyclodextrin attached with sugar group or the like.

As the alkyl derivative of cyclodextrin described above, there may be mentioned, for example, dimethyl-alpha-cyclodextrin, dimethyl-beta-cyclodextrin, dimethyl-gamma-cyclodextrin or the like.

As the hydroxyalkyl derivative of cyclodextrin described above, there may be mentioned, for example, 2-hydroxypropyl-alpha-cyclodextrin, 2-hydroxypropyl-beta-cyclodextrin, 2-hydroxypropyl-gamma-cyclodextrin or the like.

As the sulfoalkylether derivative of cyclodextrin described above, there may be mentioned, for example, sulfobutylether-alpha-cyclodextrin, sulfobutylether-beta-cyclodextrin, sulfobutylether-gamma-cyclodextrin or the like.

As the cyclodextrin attached with sugar group described above, there may be mentioned, for example, glucosyl-alpha-cyclodextrin, glucosyl-beta-cyclodextrin, glucosyl-gamma-cyclodextrin, maltosyl-alpha-cyclodextrin, maltosyl-beta-cyclodextrin, maltosyl-gamma-cyclodextrin or the like.

In the present invention, among others, sulfoalkylether derivative of cyclodextrin is preferably, and a sulfobutylether-beta-cyclodextrin is more preferably used from the point of improving the water solubility of cilostazol, These compounds are conveniently used in the form of an alkali-metal salt such as sodium salt or the like.

As, in some cases, the side effect of cyclodextrin such as renal toxicity and hemolysis might become a subject of discussion, among those sulfobutylether-beta-cyclodextrins described above, the tetra-substituted (about 4 sites in a molecule are sulfobutyl-etherized) and hepta-substituted (about 7 sites in a molecule are sulfobutyl-etherized) compounds are suitable to use, because they are almost relieved of the side effects. The hepta-substituted compound, among them, is more preferable.

The cyclodextrins and derivatives thereof, mentioned above, can be manufactured readily according to the methods already known. Also, the cyclodextrins and their derivatives on the market, for example Celdex A-100 (trade name, manufactured by Nihon Shokuhin Kako Co. Ltd.), as an alpha-cyclodextrin: Celdex B-100 (trade name, manufactured by Nihon Shokuhin Kako Co. Ltd.), as a beta-cyclodextrin; Celdex C-100 (trade name, manufactured by Nihon Shokuhin Kako Co. Ltd.), as a gamma-cyclodextrin; Celdex HP-beta-CD (trade name, manufactured by Nihon Shokuhin Kako Co. Ltd.), as a 2-hydroxypropyl-beta-cyclodextrin, and Captisol (trade name, manufactured by CyDex Inc. ) , as a sulfobutylether-beta-cyclodextrin or the like can be used appropriately.

The aqueous pharmaceutical preparation for parenteral use of the present invention may contains, as the cyclodextrin compound, a pharmacologically acceptable salt of cyclodextrin and a derivative thereof described above. As the pharmacologically acceptable salt, there may be mentioned, for example, a salt with an inorganic acid (for example, hydrochloric acid, sulfuric acid, nitric acid or the like), an organic acid (for example, carbonic acid, bicarbonic acid, succinic acid, acetic acid, propionic acid, trifluoroacetic acid or the like), an inorganic bases (for example, an alkali metal such as sodium or potassium or the like; an alkaline earth metal such as calcium or magnesium or the like) or an organic basic compound (for example, an organic amine such as triethylamine or the like; a basic amino acid such as arginine or the like).

In the present invention, an aqueous pharmaceutical preparation for parenteral use means a preparation in which the active ingredient of the present invention mentioned above is dissolved either in water or a solvent containing water. As the water, usually sterilized water, or more preferably pyrogen-free sterilized water may be used, and as the solvent containing water, those solvents which are known and have been used for manufacturing medicines may be used appropriately, exemplified by physiological saline, PBS (phosphate-buffered saline) or a Ringer solution containing lactic acid. Furthermore, the aqueous pharmaceutical preparation for parenteral use of the present invention may be prepared by drying the above mentioned aqueous pharmaceutical preparation for parenteral use, which is in the form of a solution, by means of known drying methods such as lyophilization or drying under reduced pressure to give powders or granules and then dissolving the powders or granules in water or a solvent containing water just before use.

With the aqueous pharmaceutical preparation for parenteral use of the present invention, the content of cilostazol or a pharmacologically acceptable salt thereof is, although it is difficult to define it unconditionally because it varies depending upon the application of the preparation, in a range of about 0.01 to 10 mg/mL as to the concentration of the active ingredient described above, or preferably about 0.05 to 5 mg/mL, or more preferably about 0.1 to 3 mg/mL, or most preferably about 0.5 to 2 mg/mL. Furthermore, in cases where the aqueous pharmaceutical preparation for parenteral use of the present invention is of the type of dissolving before use mentioned above, the concentration of cilostazol or a pharmacologically acceptable salt thereof means the one obtained by dissolving the said active ingredient in water or the solvent containing water.

With the aqueous pharmaceutical preparation for parenteral use of the present invention, the content of the cyclodextrin compound is, although it is difficult to define it unconditionally because it varies depending upon the application of the preparation, in a range of about 1 to 4000 mols, preferably about 1 to 200 mols, more preferably about 1 to 100 mols, and most preferably about 30 to 50 mols per 1 mol of cilostazol.

Furthermore, when sodium salt of hepta-substituted sulfobutylether-beta-cyclodextrin is used as the cyclodextrin compound, it is more preferable to adjust the concentration in the aqueous pharmaceutical preparation to about 100 to 400 mg/mL.

The aqueous pharmaceutical preparation for parenteral use of the present invention may contain an additive, in addition to the active ingredient and the cyclodextrin compound described above. As such additive, any one which is in common use in the field of technology of the aqueous pharmaceutical preparations for parenteral use may be appropriately used.

As said additive, there may be specifically mentioned a tonicity adjusting agent, a stabilizing agent, a buffer, a preservative, a chelating agent, an antioxidant, an analgesic, a solubilizing agent or the like. As the tonicity adjusting agent, there may be mentioned, for example, a sugar such as glucose, sorbitol, mannitol or the like, sodium chloride, glycerol, propylene glycol, polyethylene glycol or the like. As the stabilizing agent, there may be mentioned, for example, sodium sulfite or the like. As the buffer, there may be mentioned, for example, borate buffer, phosphate buffer, citrate buffer, tartarate buffer, acetate buffer or the like. As the preservatives, there may be mentioned, for example, a para-oxybenzoic acid ester, benzyl alcohol, a chlorocresol, phenethyl alcohol, benzethonium chloride or the like. As the chelating agent, there may be mentioned, for example, an edetic acid sodium salt, sodium citrate or the like. As the antioxidant, there may be mentioned, for example, sodium sulfite, sodium hydrogen sulfite, sodium ascorbate, sodium thiosulfate or the like. As the analgesic, there may be mentioned, for example, albumin, a polyhydric alcohol such as glycerol and propylene glycol, lidocaine hydrochloride, benzyl alcohol or the like. As the solubilizing agent, there may be mentioned, for example, dextran, polyvinylpyrrolidone, sodium benzoate, ethylenediamine, salicylamide, nicotinamide, polyoxyethylene hardened castor oil derivative or the like. Above all, addition of at least one kind of solubilizing agents selected from dextran and polyvinylpyrrolidone is preferable, because it makes it possible to improve the dissolution stability (inhibition of separation of precipitation) of the aqueous pharmaceutical preparation for parenteral use of the present invention. There is no particular restriction on the amount of solubilizing agent to be added and a necessary amount of it is appropriately selected according to the amounts of cilostazol and the cyclodextrin compound used.

The aqueous pharmaceutical preparation of the present invention may contain a pH-adjusting agent of any kind which is in common use in this field of technology to adjust pH value. The pH-adjusting agent is divided broadly into two categories: an acid and a base. Specifically, as the acid, there may be mentioned, for example, ascorbic acid, hydrochloric acid, gluconic acid, acetic acid, lactic acid, boric acid, phosphoric acid, sulfuric acid, tartaric acid, citric acid or the like, and as the base, there may be mentioned, for example, potassium hydroxide, calcium hydroxide, sodium hydroxide, magnesium hydroxide, monoethanolamine, diethanolamine, triethanolamine or the like. As the other pH adjusting agent, there may be mentioned also an aminoacid such as glycine, histidine, epsilon-aminocaproic acid or the like.

The aqueous pharmaceutical preparation for parenteral use of the present invention can be manufactured according to the ordinary method: by, for example, first dissolving the ingredients (hereinafter referred to as components) to be contained in the aqueous pharmaceutical preparation for parenteral use of the present invention into water of a solvent containing water.

The order of mixing of the components is not critical. Namely, all the components may be mixed all at once, or some components may be dissolved first in the solvent mentioned above, followed by dissolving to this the remaining components. Or, more preferably, the cyclodextrin compound is first dissolved in the solvent mentioned above, followed by the addition, to this, of cilostazol or its pharmacologically acceptable salt. Here, if desired, the additives mentioned above may be mixed and the order of mixing may be determined according to the method known per se.

Then, the resulting solution is preferably subjected to sterilization by filtration using a membrane filter or heat sterilization under pressure by means of an autoclave or heat sterilization by the method of fractional sterilization or the like. Among them, sterilization by filtration is preferably used. It is suitable that the pH of the resulting solution is in the range of about 6 to 8.

When the aqueous pharmaceutical preparation for parenteral use of the present invention is obtained as a liquid, the solution obtained after sterilization as described above may be charged and then sealed by fusing in ampoules.

According to the method of the present invention, the addition of a cyclodextrin compound increases the solubility in water of the active ingredient, which makes it possible to get a higher concentration of the active ingredient in the preparation, compared with the case in which no cyclodextrin compound is added. This leads to the advantage to be able to decrease the dose in volume necessary to be administered to give the effective dose of the active ingredient. This makes it possible, for example, to give a 10-mL one-shot injection of the aqueous pharmaceutical preparation according to the present invention, in order to administer an effective amount of the active ingredient.

In case where the aqueous pharmaceutical preparation of the present invention is made into the preparation of dissolution-before-use type preparation, a solution which contains all the components is prepared in a manner just as same as described above, sterilized as described above, and made into powders or granules according to the ordinary method. As the methods to make powders or granules from a solution, the method known per se can be employed: for example, the method such as lyophilization and drying under reduced pressure is preferably used. Among them, lyophilization is used more preferably.

As the aqueous pharmaceutical preparation for parenteral use of the present invention, there may be, more specifically, mentioned intravenous injection, intraarterial injection, subcutaneous injection, intracutaneous injection, intramuscular injection, intraspinal injection, intraperitoneal injection or the like.

There is no restriction in the administration method of the aqueous pharmaceutical preparation for parenteral use of the present invention, and it can be administered by the method appropriate to the patients from the points of their age, gender, and other conditions, together with the degree of the severity of their diseases, or the like. They may be administered, for example, separately intravenously, intraarterially, intramuscularly, intradermally, subcutaneously, intraspinally or intraperitoneally. Furthermore, the aqueous pharmaceutical preparation for parenteral use of the present invention may be mixed with a transfusion solution and administered especially intravenously as a mixture of an aqueous pharmaceutical preparation for parenteral use of the present invention and a transfusion solution. There is no particular restriction as to the transfusion solution which may be used, and the commercially available or ordinary one can be used. As the specific example of such transfusion solution, there may be mentioned glucose injection, xylitol injection, D-mannitol injection, fructose injection, physiological saline, dextran 40 injection, dextran 70 injection, amino acid injection, Ringer solution, lactic acid-Ringer solution or the like.

The dose of the aqueous pharmaceutical preparation for parenteral use of the present invention is determined according to the direction for use, age, gender and other conditions of each patient, the state of the disease or the like. It is usually preferable to select the daily dose so as to be about 1µg to 100 mg cilostazol or its pharmacologically acceptable salt per 1 kg body weight.

The aqueous pharmaceutical preparation for parenteral use of the present invention has platelet-aggregation-inhibitory action, phosphodiesterase (PDE) inhibitory action, antiulcer action, hypotensive action and antiinflammatory action and useful as, for example, antithrombotic drug, cerebral vasodilator, antiinflammatory drug, antiulcer drug, hypotensive drug, antiasthmatic drug or phosphodiesterase inhibiting drug. The aqueous pharmaceutical preparation for parenteral use of the present invention is also useful as the remedy of chronic occlusive pulmonary diseases (COPD).

### EXAMPLES

### EXAMPLE 1 to 6

Each of the following cyclodextrin compounds was dissolved in water for injection. To this, an excess amount of cilostazol was added and the mixture was shaken at 60°C for 24 hours. Then, the mixture was filtered through a membrane filter to remove insoluble materials and the amount of cilostazol in the filtrate was determined by the method of HPLC (High Performance Liquid Chromatography) to calculate the saturated concentration (solubility). Cyclodextrin compounds used were alpha-cyclodextrin (Trade Name: Celdex A-100, manufactured by NIHON SHOKUHIN KAKO Co. Ltd., beta-cyclodextrin (Trade Name: Celdex B-100, manufactured by NIHON SHOKUHIN KAKO Co. Ltd.), gamma-cyclodextrin (Trade Name: Celdex C-100, manufactured by NIHON SHOKUHIN KAKO Co. Ltd.), 6-O-alpha-maltosyl-beta-cyclodextrin (manufactured by Ensuiko Sugar Refining Co., Ltd), 2-hydroxypropyl-beta-cyclodextrin (Trade Name: Celdex HP-beta-CD, manufactured by NIHON SHOKUHIN KAKO Co. Ltd), hepta-substituted sulfobutylether-beta-cyclodextrin sodium salt (hereinafter abbreviated as SBE7-beta-CD)(Trade Name: Captisol, manufactured by CyDex Inc.).

Also, as a Reference Example, the solubility of cilostazol without addition of cyclodextrin compounds was determined similarly.

Table 1 shows the results.

**Table 1**

| | cyclodextrin compound | concentration (g/L) | saturated solubility of cilostazol (mg/L) |
|---|---|---|---|
| EXAMPLE 1 | alpha-cyclodextrin | 150 | 32 |
| EXAMPLE 2 | beta-cyclodextrin | 15 | 105 |
| EXAMPLE 3 | delta-cyclodextrin | 200 | 83 |
| EXAMPLE 4 | 6-O-alpha-maltosyl-beta-cyclodextrin | 200 | 120 |
| EXAMPLE 5 | 2-hydroxypropyl-beta-cyclodextrin | 40 | 212 |
| EXAMPLE 6 | SBE7-beta-CD | 300 | 1150 |
| REFERENCE EXAMPLE | | - | 3.5 |

### FORMURATION EXAMPLE 1

SBE7-beta-CD (Trade Name: Captisol, Manufactured by CyDex Inc.) was dissolved in water for injection, and to this were further added cilostazol, sodium bisulfite and methyl para-oxybenzoate, adjusting the concentrations of each ingredient to the values shown below. The parenteral solution was filtered through 0.22-µm membrane filter and the filtrate charged and sealed in glass ampoules 10 mL each to give the final product.

This final product showed no change in its appearance, when it was left standing at ordinary temperature for one year.

| | |
|---|---|
| SBE7-beta-CD | 300 g/L |
| cilostazol | 1 g/L |
| sodium bisulfite | 10 g/L |
| methyl para-oxybenzoate | 20 g/L |

### FORMURATION EXAMPLE 2

A parenteral solution, of which the prescription is shown below, was produced by the method similar to that used for the production of Preparation Example 1. This preparation was, similar to those in Preparation Example 1, also stable for one year at ordinary temperature.

| | |
|---|---|
| SBE7-beta-CD | 300 g/L |
| cilostazol | 1 g/L |
| dextran 40 | 50 g/L |
| sodium bisulfite | 10 g/L |
| methyl para-oxybenzoate | 20 g/L |

### INDUSTRIAL APPLICABILITY

In the aqueous pharmaceutical preparation for parenteral use of the present invention, the slightly soluble active ingredients such as cilostazol or a pharmacologically acceptable salt thereof are made more soluble in water by the addition of cyclodextrin compounds. As the result, the aqueous pharmaceutical preparation for parenteral use of the present invention is excellent in the liquid state stability with time, and, there occurs, for example, practically no separation of crystals of the active ingredient even in the storage of a long-term. Thus, the present invention can provide us not only an aqueous pharmaceutical preparation which has to be dissolved before use, but a ready-to-use aqueous pharmaceutical preparation. Furthermore, the present invention solves the problem which comes from the fact that there have only been oral preparations, and can provide patients suffering from acute myocardial or cerebral infarctions remedies ready to administer and of immediate action. Thus, the preparation of the present invention is really useful especially for treating patients in the acute phases of their diseases.

## Claims

1. A cilostazol aqueous pharmaceutical preparation for parenteral use, which comprises cilostazol or a pharmacologically acceptable salt thereof as an active ingredient and a cyclodextrin compound.

2. A cilostazol aqueous pharmaceutical preparation for parenteral use as claimed in claim 1, wherein cilostazol or a pharmacologically acceptable salt thereof and a cyclodextrin compound are dissolved in either water or a solvent containing water.

3. A cilostazol aqueous pharmaceutical preparation for parenteral use as claimed in claim 2, wherein the preparation contains cilostazol or a pharmacologically acceptable salt thereof in a concentration from 0.01 to 20 mg/mL.

4. A cilostazol aqueous pharmaceutical preparation for parenteral use as claimed in claim 3, wherein it contains 1 to 4000 moles of a cyclodextrin compound per 1 mole of cilostazol.

5. A cilostazol aqueous pharmaceutical preparation for parenteral use as claimed in claim 1, wherein the cyclodextrin compound is a sulfoalkylether derivative of cyclodextrin.

6. A cilostazol aqueous pharmaceutical preparation for parenteral use as claimed in claim 5, wherein the cyclodextrin sulfoalkylether is in the form of an alkali metal salt thereof.

7. A cilostazol aqueous pharmaceutical preparation for parenteral use as claimed in claim 5, wherein the cyclodextrin sulfoalkylether derivative is a sulfobutylether-beta-cyclodextrin alkali-metal salt.

8. A cilostazol aqueous pharmaceutical preparation for parenteral use as claimed in claim 5, wherein the cyclodextrin sulfoalkylether is a hepta-substituted sulfobutylether-beta-cyclodextrins alkali-metal salt.

9. A cilostazol aqueous pharmaceutical preparation for parenteral use as claimed in claim 8, wherein the preparation contains cilostazol or a pharmacologically acceptable salt thereof in a concentration from 0.01 to 20 mg/mL.

10. A cilostazol aqueous pharmaceutical preparation for parenteral use as claimed in claim 9, wherein the preparation contains from 1 to 4000 moles of a cyclodextrin compound per 1 mole of cilostazol.

11. A cilostazol aqueous pharmaceutical preparation for parenteral use as claimed in claim 10, wherein the preparation contains cilostazol or a pharmacologically acceptable salt thereof in a concentration of 0.05 to 5 mg/mL and heptasubstituted sulfobutylether-beta-cyclodextrin sodium salt in a concentration of 100 to 400 mg/mL.

12. A cilostazol aqueous pharmaceutical preparation for parenteral use as claimed in claim 11, wherein the preparation contains, furthermore, at least one of dissolution auxiliaries selected from dextran and polyvinylpyrrolidone.

13. Use of a cyclodextrin compound for increasing the water solubility of cilostazol or a pharmacologically acceptable salt thereof.

14. A symptom ameliorant of an acute myocardial or a cerebral infarction in their acute phases, which comprises the aqueous pharmaceutical preparation containing cilostazol or pharmacologically acceptable salt thereof as claimed in any of claims 1 to 11.

15. A symptom ameliorant of an acute myocardial or a cerebral infarction as claimed in claim 14, which comprises furthermore at least one of dissolution auxiliaries selected from dextran and polyvinylpyrrolidone.

16. A method to ameliorate a symptom in the acute phase of acute myocardial or a cerebral infarction, which comprises administering to a patient via vein an aqueous pharmaceutical preparation of cilostazol as claimed in any of claims 1 to 11.
